# EUROPEAN PATENT SPECIFICATION

(11) **EP 1 104 407 B1**
(45) Date of publication and mention of the grant of the patent: **01.12.2004**
(21) Application number: 00939699.5
(22) Date of filing: 08.06.2000
(51) Int. Cl.: C07D 233/54, A61K 31/33, A61P 43/00, C07D 409/06, C07D 263/28, C07D 413/12, C07D 233/84, C07D 233/10, C07D 405/06, C07D 401/06, C07D 403/06, C07D 495/04

(54) **COMPOUNDS AS SELECTIVE AGONISTS AT ALPHA 2B OR 2B/2C ADRENERGIC RECEPTORS**
VERBINDUNGEN ALS SELEKTIVE AGONISTEN DER ALPHA 2B ODER 2B/2C ADRENERGISCHE REZEPTOREN
COMPOSES UTILISABLES COMME AGONISTES SELECTIFS DES RECEPTEURS ADRENERGIQUES ALPHA 2B OU 2B/2C

(30) Priority: 10.06.1999 US 329752
(43) Date of publication of application: 06.06.2001
(73) Proprietor: Allergan, Inc., Irvine, CA 92612 (US)
(72) Inventor: CHOW, Ken, Newport Coast, CA 92657 (US); GIL, Daniel, W., Corona Del Mar, CA 92625 (US); BURKE, James, A., Santa Ana, CA 92705 (US); HARCOURT, Dale, A., San Clemente, CA 92672 (US); GARST, Michael, E., Newport Beach, CA 92660 (US); WHEELER, Larry, A., Irvine, CA 92612 (US); MUNK, Stephen, A., Northville, MI (US)
(74) Representative: HOFFMANN - EITLE
(86) International application number: PCT/US2000/015795
(87) International publication number: WO 2001/000586

(56) References cited:
- EP-A- 0 024 829
- WO-A-97/12874
- WO-A-99/18075
- WO-A-99/28300
- US-A- 5 621 113
- US-A- 5 750 720
- CHEMICAL ABSTRACTS, vol. 112, no. 15, 9 April 1990 (1990-04-09) Columbus, Ohio, US; abstract no. 139033n, KIHARA, NORIAKI ET AL: "Preparation of imidazole derivatives as drugs." XP002151513 -& DATABASE CHEMICAL ABSTRACTS [Online] CA 112:139033, XP002151515 & JP 01 242571 A (MITSUI PETROCHEMICAL INDUSTRIES, LTD.)
- CHEMICAL ABSTRACTS, vol. 100, no. 24, 11 June 1984 (1984-06-11) Columbus, Ohio, US; abstract no. 197710, LAINE, ENSIO ET AL: "Physical studies of detomidine hydrochloride, a new drug substance." XP002151514 & ACTA PHARM. SUEC., vol. 20, no. 6, - 1983 pages 451-460,
- YOSHIYA AMEMIYA ET AL: "Synthesis and alpha-adrenergic activities of 2- and 4-substituted imidazoline and imidazole analogues" JOURNAL OF MEDICINAL CHEMISTRY., vol. 35, no. 4, - 1992 pages 750-755, XP002151512 AMERICAN CHEMICAL SOCIETY. WASHINGTON., US ISSN: 0022-2623

## Description

### 1. Field of the Invention

The present invention is directed to a method of treating glaucoma or elevated intraocular pressure and other diseases with substantially reduced cardiovascular or sedative side effects by administering to mammals including humans, compounds which are selective agonists of the α2B alone or α2B and α2C adrenergic receptor subtypes and which lack substantial activity at the α2A receptor subtype. The present invention is also directed to novel compounds and pharmaceutical compositions adapted for administering said compounds to mammals, including humans.

### 2. Brief Description of the Prior Art

Compounds which have adrenergic activity are well known in the art, and are described in numerous United States and foreign patents and in scientific publications. It is generally known and accepted in the art that adrenergic activity is useful for treating animals of the mammalian species, including humans, for curing or alleviating the symptoms and conditions of numerous diseases and conditions. In other words, it is generally accepted in the art that pharmaceutical compositions having an adrenergic compound or compounds as the active ingredient are useful for treating glaucoma, chronic pain, nasal congestion, high blood pressure, congestive heart failure and inducing anesthesia.

The two main families of adrenergic receptor are termed alpha adrenergic receptors and beta adrenergic receptors in the art, and each of these two families is known to have subtypes, which are designated by letters of the alphabet, such as α2A, α2B. See the article by Bylund et al, *Pharmacol Rev.* **46,** pp. 121-136(1994).

### SUMMARY OF THE INVENTION

It has been discovered in accordance with the present invention that adrenergic compounds which act selectively, and preferably even specifically as agonists of the α2B or α2B / α2C (hereinafter referred to as α2B or α2B/2C) receptor subtypes in preference over the α2A receptor subtype, possess desirable therapeutic properties associated with adrenergics but without having one or more undesirable side effects such as changes in blood pressure or sedation. For the purposes of the present invention, a compound is defined to be a specific or at least selective agonist of the α2B or α2B/2C receptor subtype(s) if the compound is at least approximately ten times more potent as an agonist at either the α2B and α2C or both receptor subtypes than at the α2A receptor subtype, or if the difference in the compound's efficacy at the α2B and α2B/2C receptor relative to the α2A receptor is greater than 0.3 and its efficacy at the α2A receptor is ≤ 0.4.

Accordingly, the present invention relates to methods of treating animals of the mammalian species, including humans, with a pharmaceutical composition comprising one or more specific or selective α2B or α2B/2C adrenergic agonist compounds as the active ingredient, for treatment of the many diseases or conditions against which alpha adrenergic compounds are useful, including without limitation glaucoma, reducing elevated intraocular pressure, chronic pain, diarrhea, and nasal congestion. In addition, the compounds of this invention are useful for treating muscle spasticity including hyperactive micturition, diarrhea, diuresis, withdrawal syndromes, pain including neuropathic pain, neurodegenerative diseases including optic neuropathy, spinal ischemia and stroke, memory and cognition deficits, attention deficit disorder, psychoses including manic disorders, anxiety, depression, hypertension, congestive heart failure, cardiac ischemia and nasal congestion.

The present invention is also directed to the pharmaceutical compositions used in the above-noted methods of treatment.

The present invention particularly covers methods for treating diseases and conditions where adrenergic compounds are effective for treatment, but their use is limited because of their generally known side effects.

### DETAILED DESCRIPTION OF THE INVENTION

Compounds which are used in the pharmaceutical compositions and methods of treatment of the present invention are selective or specific agonists of the α2B or α2B/2C adrenergic receptor subtypes, in preference over the α2A receptor subtype. In accordance with the present invention, a compound is considered a selective α2B or α2B/2C agonist if that compound's difference in efficacy as an agonist of the α2B or α2B/2C receptor subtype(s) compared to the α2A receptor subtype is greater than 0.3 and its efficacy at the α2A receptor subtype is ≤ 0.4 and/or it is at least approximately 10 times more potent. Preferably, the compounds utilized in accordance with the present invention are specific agonists of the α2B or α2B/2C receptor subtypes. Specifically, in this regard, a specific agonist is defined in the sense that a specific α adrenergic agonist does not act as an agonist of the α2A receptor subtype to any measurable or biologically significant extent.

A set of agents has been discovered that are functionally selective for the α2B or α2B/2C - subtypes of said adrenergic receptors. This preferential activity can be determined in a variety of functional assays such as Cyclic AMP Production, Shimizu et al, *J. Neurochem.* **16,** pp. 1609-1619 (1969); R-SAT (Receptor Selection and Amplification Technology), Messier et al, *Pharmacol*. *Toxicol.* **76,** pp. 308-311(1995) and the Cytosensor microphysiometer, Neve et al, *J*. *Biol. Chem*. **267,** pp. 25748-25753, (1992) using cells that naturally express individual subtypes or have had one of the subtypes introduced. The cells or recombinant receptors used should be human or from a species that has been shown to have a similar pharmacology. In the study below, the RSAT assay on cells that have been transiently transfected with the human α2A (c10 gene), rat α2B (RNG gene) and human α2C (c4 gene) receptors was used. The rat α2B receptor has been shown to have a pharmacology that corresponds to the human α2B receptor (see, for example, Bylund et al., *Pharmocol, Rev.* **46,** pp. 127-129(1994)).

In the treatment of glaucoma, particularly, topical administration may be used. Any common topical formulation such as a solution, suspension, gel, ointment, or salve and the like may be applied to the eye in glaucoma and dermally to treat other indications. Preparation of such topical formulations are well described in the art of pharmaceutical formulations as exemplified, for example, by Remington's Pharmaceutical Science, Edition 17, Mack Publishing Company, Easton, Pennsylvania.

If the drug is to be administered systemically, it may be confected as a powder, pill, tablet or the like or as a syrup or elixir for oral administration. For intravenous, intraperitoneal, intrathecal or epidural administration, the compound will be prepared as a solution or suspension capable of being administered by injection. In certain cases, it may be useful to formulate these compounds in suppository or as an extended release formulation, including the dermal patch form, for deposit on or under the skin or for intramuscular injection.

Treatment of glaucoma or any other indications known or discovered to be susceptible to treatment by adrenergic compounds will be effected by administration of therapeutically effective dose of one or more compounds in accordance with the instant invention. A therapeutic concentration will be that concentration which effects reduction of the particular condition, or retards its expansion. In certain instances, the drug potentially could be used in a prophylactic manner to prevent onset of a particular condition. A given therapeutic concentration will vary from condition to condition and in certain instances may vary with the severity of the condition being treated and the patient's susceptibility to treatment. Accordingly, a given therapeutic concentration will be best determined at the time and place through routine experimentation. However, it is anticipated that in the treatment of, for example, glaucoma, that a formulation containing between 0.001 and 5 percent by weight, preferably about 0.01 to 3% will usually constitute a therapeutically effective concentration. If administered systemically, an amount between 0.001 and 50 mg per kg, preferably between 0.001 and 10 mg per kg body weight per day, but most preferably about 0.01 to 1.0 mg/kg, will effect a therapeutic result in most instances.

Because the α2B and α2B/2C specific selective agonist compounds lack substantial α2A side effects, treatments of diseases or conditions with such compounds in accordance with the present invention is advantageous, particularly when the treatment is directed to a human having cardiovascular problems.

The general structures of exemplary specific α2B and α2C agonist or selective α2B and α2B/2C agonist adrenergic compounds which are used in the pharmaceutical compositions and methods of treatment of the present invention are provided by general formulas.

In the following the synthesis of the claimed compounds is illustrated

### Example S

Procedure for the preparation 4(5)-(4a-methyl-2,3,4,4a,5,6,7,8-octahydronaphthalen-2-ylmethyl)-1H-imidazole, but-2-enedioic acid salt :

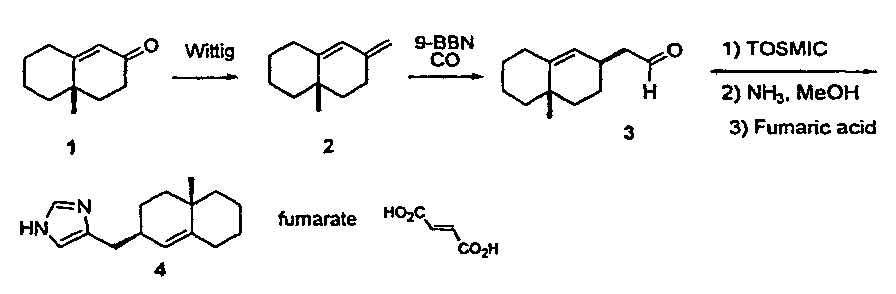

### Procedure -

Methyl triphenylphosphonium bromide (2.75 g, 7.70 mmol) was suspended in 50 mL of diethyl ether. At -10 °C, *n*BuLi (3.08 mL, 7.70 mmol, 2.5M soln in hexanes) was added. This mixture was stirred for 35 m before cooling to -70 °C. A solution of (*R*)-(+)-4,4a,5,6,7,8-hexahydro-4a-methyl-2(3*H*)-naphthalenone (**1**) (1.0 g, 6.09 mmol) in 15 mL of ether was added *via* syringe. This mixture was warmed to 0 °C over 30 m and the stirred at rt for another 30 m. The solution was washed with brine (2 x 20 mL) dried over MgSO₄, filtered and the solvent was removed. Chromatography on SiO₂ with hexanes gave 0.82 g (83%) of the diene 2 as a clear colorless oil.

This hydroboration procedure follows that by Brown, H. C. *et. al*. *J. Am. Chem. Soc*. **1969,** *91*, 2144. To a solution of the diene **2** (750 mg, 4.63 mmol) in 20 mL of THF was added 9-BBN (11.8 mL, 5.9 mmol, of a 0.5 M soln. in THF) at 0 °C. This was warmed to rt after 30 m and allowed to react at rt for 1 h. Dry MeOH (3.75 mL, 15.0 mmol as a 4.0 M soln in THF) was added to a stirred solution of LiAlH₄ (5.04 mL, 5.04 mmol, 1.0 M in ether) to form LiAlH(OMe)₃. The borane was added to this alkoxy aluminum hydride *via* syringe. After 10 m at rt, carbon monoxide was bubbled through the solution for 20 m. Phosphate buffer (25 mL, pH 7.0 was added followed by H₂O₂ (10 mL, 30% soln) and this was stirred for 30 m. After a typical extraction process the oil was purified by chromatography on SiO₂ with 5 to 10% EtOAc:Hx to yield the colorless aldehyde **3** as the major product 455 mg, (51%).

This preparation followed the protocol by Home, D. A.; Yakushijin, K.; Büchi, G. *Heterocycles*, **1994,** *39*, 139. A solution of the above aldehyde **3** (450 mg, 2.34 mmol) in EtOH (8 mL) was treated with tosylmethyl isocyanide (TosMIC) (430 mg, 220 mmol) and NaCN (∼15 mg, cat) at rt for 20 m. The solvent was removed in vacuo and the residue dissolved in MeOH saturated with NH₃ (10 mL). The solution was heated in a resealable tube at 110°C for 6-12 h. The material was concentrated and purified by chromatography on SiO₂ with 5% MeOH (sat. w/ NH₃) :CH₂Cl₂ to give the imidazole as a thick glass 193 mg (36%).

The imidazole was purified further by stirring in THF or MeOH with an equimolar amount of fumaric acid at rt for 10 m. The solvent was removed and the salt recrystallized by dilution in THF and tituration with ether:hexanes for a 70-80% recovery of pure fumarate 4 (S).
¹H NMR (500 MHz, DMSO-d₆ w/ TMS): δ 7.73 (s, 1 H), 6.83 (s, 1 H), 6.60 (s, 2 H), 5.12 (s, 1 H), 2.45-2.44 (m, 2 H), 2.30 (brs, 1 H), 2.12 (brs, 1 H), 1.91-1.88 (m, 1 H), 1.73-1.71 (m, 1 H), 1.56-1.46 (m, 5 H), 1.30-1.09 (series of m, 4 H), 1.01 (s, 3 H)
¹³C (125 MHz, DMSO-d₆ w/ TMS) : δ 167.0, 143.5, 134.8, 134.5, 128.7, 123.7, 118.2, 42.3, 36.7, 35.0, 32.8, 32.5 (2C), 28.4, 25.9, 24.4, 22.3.

### Example T-1

Procedure for the preparation 4(5)-(3-methyl-cyclohex-2-enylmethyl)-1H-imidazole, but-2-enedioic acid salt :

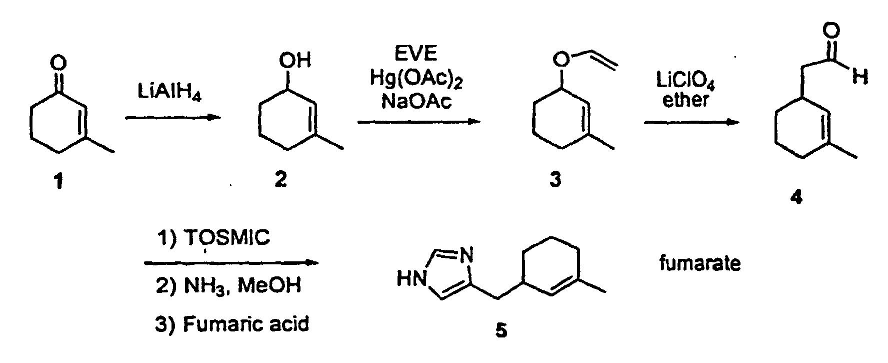

### Procedure -

A solution of 3-methyl-2-cyclohexen-1-one (1) (5g, 45.4 mmol) in 25 mL of ether was added dropwise *via* an addition funnel to a solution of LiAlH₄ (45 mL, 1M in THF) in ether (100 mL) at -10 °C. After 1 h the mixture was carefully quenched with NH₄Cl (10 mL) and treated with 10% HCl (7 mL). The organic layer was extracted with ether (3 x 70 mL), dried over MgSO₄, filtered and concentrated. The residue was purified by chromatography by elution with 20% EtOAc:Hx to give 2, a clear colorless alcohol, 4.46 g (88%).

A solution of alcohol 2 (1.68 g, 15 mmol) in ethyl vinyl ether (38 mL) was treated with Hg(OAc)₂ (3.2 g, 10 mmol) and NaOAc (410 mg, 5 mmol) at 35 °C for 4 h. The mixture was poured onto 5% KOH solution (15 mL), diluted with ether and extracted with hexanes. The organic layer was dried over Na₂SO₄, filtered and concentrated. The crude residue was used in the next step without further purification.

According to the procedure by Greico, P. A.; *et al*, *J. Am Chem. Soc.* **1991,** *113*, 5488, a 3M solution of LiClO₄ (16 g, 150 mmol) in 50 mL of ether was treated with the crude vinyl ether 3 at rt for 30 m. The entire mixture was poured onto sodium bicarbonate solution (150 mL). After extraction of the aldehyde **4** with ether, the organic layer was dried over MgSO₄, filtered, and concentrated under reduced pressure. The crude residue was purified by chromatography on SiO₂ with EtOAc:Hx or submitted to the Büchi protocol as described above for the formation of the imidazole-fumarate **5** (8% from **6** to free base of **5**).
^{**1**}**H NMR** (500 MHz, d⁶-DMSO w/ TMS) : δ 7.71 (s, I H), 6.82 (s, 1 H), 6.61 (s, 2 H), 5.27 (s, 1 H), 2.46-2.32 (series of m, 3 H), 1.85 (brs, 2 H), 1.60 (s, 3 H), 1.35-0.86 (series of m, 4 H)
^{**13**}**C** (125 MHz, DMSO-d₆ w/ TMS) : δ 167.3, 134.9, 134.5, 125.5, 118.1, 35.5, 32.6, 30.1, 28.5, 24.0, 21.4.

### Example T-2

4(5)-(3,5,5-trimethyl-cyclohex-2-enylmethyl)-1H-imidazole, but-2-enedioic acid salt is prepared by substituting isophorone in the method of T-1

### Example T-3

4(5)-(3-methyl cyclopent-2-enylmethyl)-1H-imidazole, but-2-enedioic acid salt is prepared by substituting 3-methyl-2-cylopenten-1-one in the method of T-1

### Example U-1

Procedure for the preparation 4(5)-cyclohex-2-enylmethyl-1H-imidazole, but-2-enedioic acid salt:

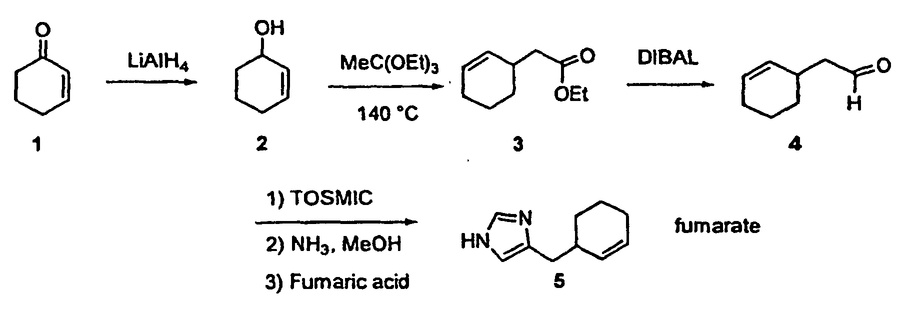

### Procedure-

A solution of cyclohexenone **(1)** (2.88 g, 30 mmol) in hexanes at -78 °C was treated with DIBAL (30 mL, 1.0 M in cyclohexane). After 25 m, MeOH (7 mL) was added and the mixture was warmed to rt. A saturated solution of Rochelle's salt was added followed by dilution with ether (100 mL). The organic layer was separated, dried over MgSO₄, filtered and concentrated under vacuum. The product was purified by chromatography on SiO₂ with 20% EtOAc:Hx to give a clear colorless alcohol **2**, 2.0 g (68%).

A solution of the above alcohol 23 (2.0 g, 20.4 mmol) in triethyl orthoacetate (30 mL) and propionic acid (-0.025 mL, cat) was heated to remove ethanol. After the ethanol was removed heating was continued at 145 °C for 1 h. The triethyl orthoacetate was removed by simple distillation. After the residue cooled to rt the product was purified by chromatography on SiO₂ with 5% ether:Hx to give ester **3** as a clear colorless oil 1.08g (∼31%).

A solution of the above ethyl ester 3 (1.0 g, 5.9 mmol) was dissolved in hexanes (50 mL) and cooled to -78 °C. A solution of DIBAL (5.8 mL 1.0 M in cyclohexane) was added dropwise. After 15 m, diethyl ether (50 mL) was added and the mixture was stirred with Rochelle's salt solution (25 mL) for 10 m. The organic layer was separated, dried and filtered. Chromatography on SiO₂ with 7% Et₂O:Hx delivered the aldehyde as a clear colorless oil, 0.52g (74%). The aldehyde 4 was subjected to the Büchi protocol as described above. The fumarate salt of the imidazole 5 (U-l) was obtained in three steps (25% overall).
^{**1**}**H NMR** (500 MHz, DMSO-d₆ w/ TMS) : δ 7.67 (s, 1 H), 6.80 (s, 1 H), 6.60 (s, 2 H), 5.66-5.54 (m, 2 H), 2.52-2.42 (m, 2 H), 2.34 (brs, 1 H), 1.93 (s, 2 H), 1.66 (brs, 2 H), 1.46-1.43 (m, 1 H), 1.22-1.16 (m, 1 H)
^{**13**}**C** (125 MHz, DMSO-d₆ w/ TMS) : δ 166.3, 134.3, 134.2, 131.2, 126.9, 118.1, 96.5, 35.0, 32.5, 28.4, 24.8, 20.7.

### Example U-2

4(5)-(4-methyl-cyclohex-2-enylmethyl)-1H-imidazole, but-2-enedioic acid salt is prepared by substituting 6-methyl-2-cyclohexen-1-one in the method of U-1

### Example V

Procedure for the preparation of 2-(1H-Imidazole-4(5)-ylmethyl)-cyclohexanone, but-2-enedioic acid salt:

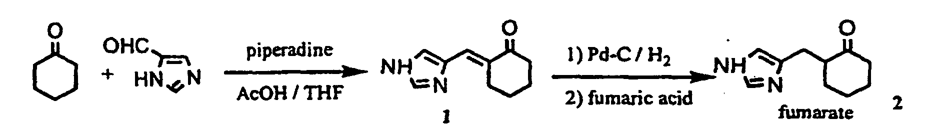

### Procedure -

To the 4(5)-imidazolecarboxaldehyde (2.52 g, 26.23 mmol) suspended in cyclohexanone (25.74 g, 262..25 mmol) under argon added the piperadine (0.56 g, 6.56 mmol) and acetic acid (0.52 g, 8.65 mmol). After heating at reflux for 16 h. the cyclohexanone was removed by kugelrohr. Chromatography on SiO₂ with 5-10% MeOH (saturated with NH₃): CH₂Cl₂ gave 4.07 g (88%) of unsaturated imidazole **1** as an oil.

The unsaturated imidazole **1** (1.02 g, 5.81 mmol) in MeOH (40 ml) containing palladium (10 wt. % on activated carbon) (0.15 g) was hydrogenated at 1 atmosphere pressure of H₂. After 16 h the palladium was filtered off and the filtrate was concentrated at reduced pressure. The imidazole was recrystallized by stirring in MeOH with an equimolar amount of fumaric acid until all solids had disappeared followed by the addition of a small amount of diethyl ether and cold storage. The title compound **2** (V) 0.80 g (48%) was recovered as white crystals.
^{**1**}**H NMR** (300 MHz, CDCl₃ w/ TMS) : δ 9.5-6.5 (vbs, 3H), 7.71(s, 1H), 6.80 (s, 1H), 6.60 (s, 2H), 2.91(dd, J = 14.8 Hz, J = 5.4 Hz, 1H), 2.75-2.60 (m, 1H), 2.42-2.28 (m, 2H), 2.27-2.17 (m, 1H), 2.02-1.89 (m, 2H), 1.78-1.68 (m, 1H), 1.68-1.45 (m, 2H), 1.32-1.17 (m, 1H)
^{**13**}**C NMR** (75MHz, DMSO-d₆ w/ TMS) : δ 211.6, 166.6, 134.4, 134.2, 133.8, 117.4, 49.7, 41.4, 33.1, 27.5, 25.8, 24.3.

### Example W-1

Procedure for the preparation of 4(5)-(3,4-Dimethyl-cyclohex-3-enylmethyl)-1H-imidazole, but-2-enedioic acid salt:

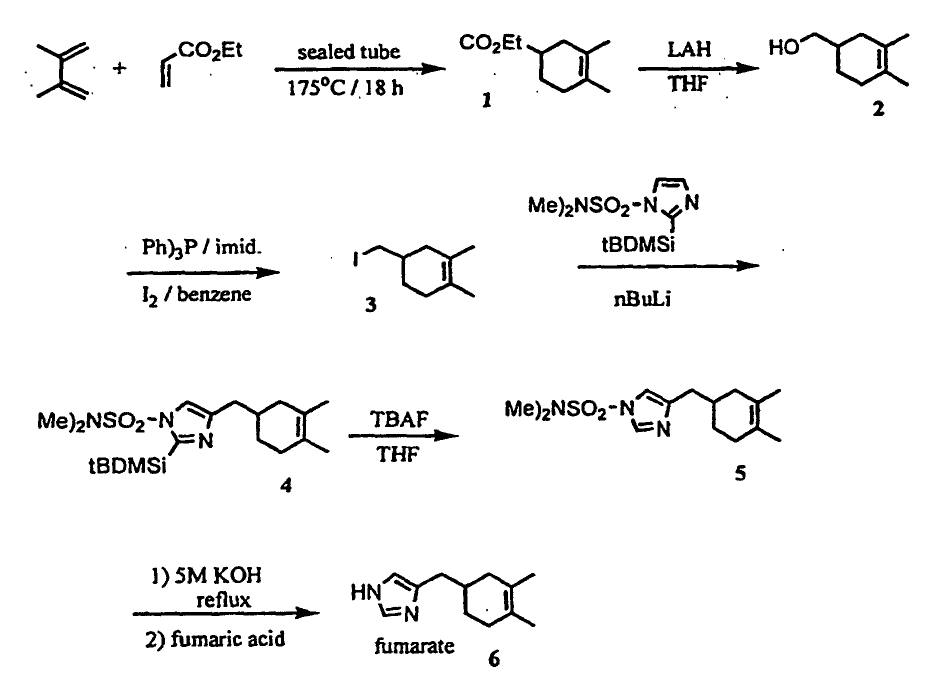

### Procedure -

2,3-Dimethyl-1,3-butadiene (10.16 g, 123.72 mmol), ethyl acrylate (11.06 g, 110.47 mmol) and hydroquinone (0.12 g, 1.11 mmol) were heated with stirring at 165°C in a sealed tube for 16 h and then at 205°C for an additional 4 h. Kugelrohr distillation of the resulting residue at 150°C and 0.5 torr gave 14.11 g (70%) of cyclohexene ester **1** as an oil in the 20°C bulb. To a solution of the ester **1** (13.62 g, 72.32 mmol) in anhydrous THF (200 ml) at -78°C under argon added the LiAlH₄ (54.30 ml, 1 M in diethyl ether). This mixture was stirred for 1 h at 20°C and then quenched at 0°C by the careful, consecutive addition of H₂O (2.06 ml), NaOH (2.06 ml of a 15% aqueous solution), and an additional portion of H₂O (6.18 ml). The solids were filtered off and the filtrate was concentrated under reduced pressure. Kugelrohr distillation of the resulting residue at 150-180°C and 0.5 torr gave 9.98 g (98%) of the alcohol **2** as a colorless volatile oil in the 0°C bulb. To a solution of triphenyl phosphine (27.13 g, 103.45 mmol), and imidazole (7.04g, 103.45 mmol) in anhydrous benzene (450 ml) under argon was added the I₂ (22.75 g, 89.61 mmol) in benzene (170 ml) over a period of 10 minutes with rapid mechanical stirring. After an additional 10 minutes the alcohol 2 (9.23 g, 65.89 mmol) in benzene (100 ml) was added to this rapidly stirring mixture over a period of 5 minutes. After 2 h the reaction was diluted with hexanes (800 ml) and the solids were filtered off. The organics were washed with 3 portions of H₂O (800 ml), dried (MgSO₄), filtered and concentrated under reduced pressure. The residual solids were filtered off and the resulting oil was purified by kugelrohr distillation at 200°C and 0.5 torr to give 11.99 g (73%) of the iodide **3** as a pale oil in the 0°C bulb. To a solution of the previously described 1- N-(dimethylsulfamoyl)-2-tert-butyldimethylsilyl imidazole (4.34 g, 15.00 mmol) in anhydrous THF (50 ml) at -78°C under argon was added n-butyllithium (5.76 ml, 2.5 M in hexanes). This mixture was stirred for 10 minutes at -10°C and then cooled to -20°C before adding the iodide **3** (3.00 g, 12.00 mmol) in THF (25 ml) dropwise via cannula. The resulting solution was stirred for 16 h at 20°C, then quenched with saturated aqueous NaHCO₃ and concentrated under reduced pressure. The residues were taken up in diethyl ether and washed consecutively with H₂O and brine, dried (MgSO4) and concentrated. Subsequent purification by chromatography on SiO₂ with 5-10% EtOAc:hexanes gave 0.89 g (15%) of the imidazole **4** as a pale oil. To a solution of imidazole **4** (0.89 g, 2.17 mmol) in anhydrous THF (25 ml) under argon was added tetrabutylammonium fluoride (2.38 ml, 1 M in THF) and the resultant solution was stirred for 1 h at 20°C. The mixture was concentrated under reduced pressure and the residues were taken up in diethyl ether and washed consecutively with saturated aqueous NaHCO₃ and brine, dried (MgSO₄) and concentrated. The residues were purified by chromatography on SiO₂ with 50% EtOAc:hexanes to give 0.56 g (87%) of the imidazole **5** as a pale oil. To a solution of **5** (0.53 g, 1.77 mmol) in MeOH (5 ml) was added aqueous KOH (15 ml of a 5M solution) and the mixture was heated at reflux for 32 h. The mixture was concentrated under reduced pressure, diluted with H₂O (5 ml) and extracted exhaustively with CHCl₃. The combined organic fractions were washed consecutively with H₂O and brine, dried (MgSO₄) and concentrated under reduced pressure. The imidazole was recrystallized by stirring in MeOH with an equimolar amount of fumaric acid until all solids had disappeared followed by the addition of a small amount of diethyl ether. The title compound **6** (W-1) 0.27 g (57%) was recovered as pale crystals.
^{**1**}**H** NMR (300 MHz, DMSO-d₆ w/TMS) :δ 10.3-8.8 (vbs, 3 H), 7.88 (s, 1H), 6.89 (s, 1H), 6.59 (s, 2H), 2.48 (d, J = 6.7 Hz, 2 H), 2.00-1.70 (m, 4 H), 1.70-1.57 (m, 2 H), 1.56 (s, 3 H), 1.54 (s, 3 H), 1.21-1.04 (m, 1 H))
^{**13**}**C NMR** (75MHz, DMSO-d₆ w/ TMS) : δ 166.7, 134.4, 134.1, 133.4, 124.8, 124.3, 117.9, 37.6, 34.1, 32.2, 31.1, 28.7, 19.0, 18.7.

### Example W-2

4(5)-Cyclohex-3-enylmethyl-1H-imidazole, but-2-enedioic acid salt is prepared by substituting 3-cyclohexene-1-methanol in the method of W-1

### Example X-1

Procedure for the preparation of 4(5)-(4-Methyl-cyclohex-3-enylmethyl)-1H-imidazole, but-2-enedioic acid salt:

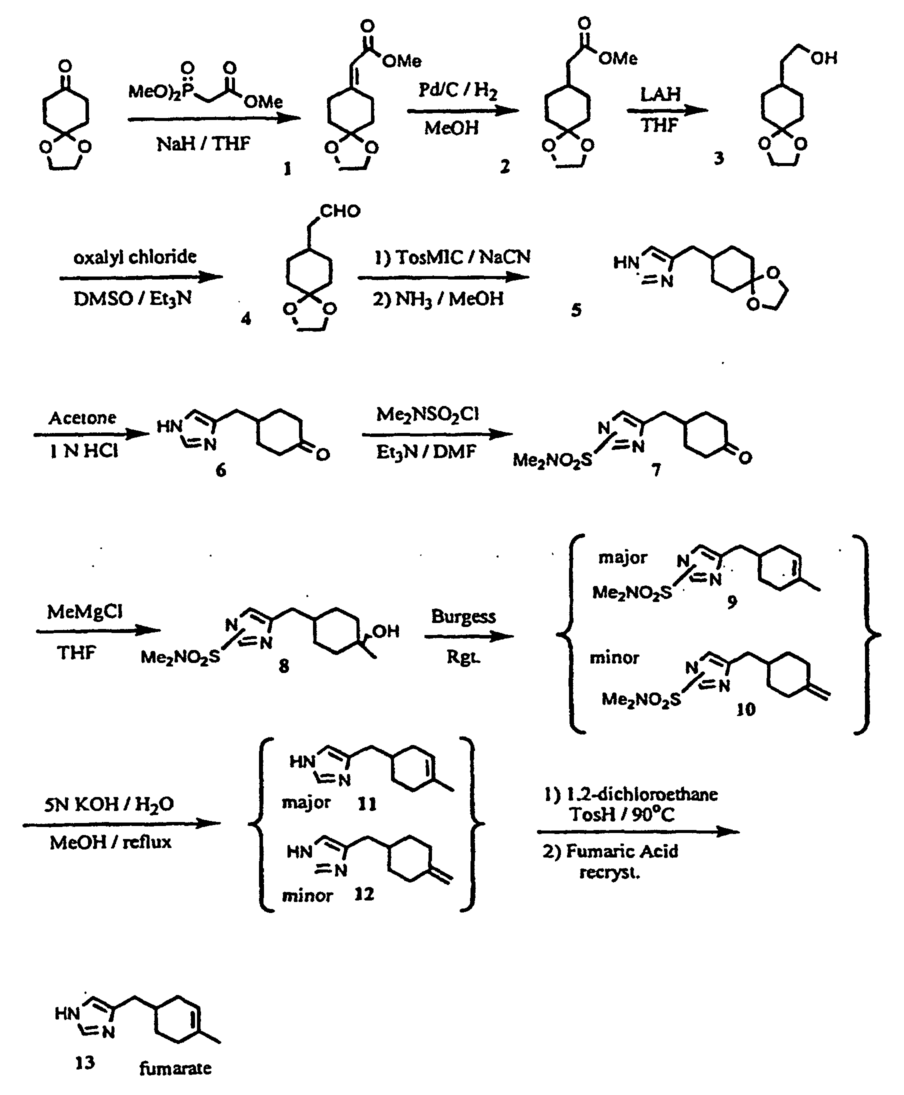

### Procedure -

To a slurry of NaH (60% in oil) (6.92 g, 288.28 mmol) in anhydrous THF (1500 ml) at 0°C under argon with vigorous mechanical stirring added the trimethyl phosphonoacetate (52.50 g, 288.28 mmol) dropwise. Stirred this mixture an additional 30 minutes before adding the 1,4-cyclohexanedione mono-ethylene ketal (40.93 g, 262.07 mmol) in THF (170 ml) dropwise. The mixture was stirred an additional 18 h at 20°C and then concentrated under reduced pressure. This residue was taken up in diethyl ether (1000 ml) and washed consecutively with H₂O and brine, dried (MgSO₄), filtered and concentrated to give 60.08 g (98%) of the unsaturated ester **1** which was carried on without further purification. To a solution of unsaturated ester **1** in EtOAc (500 ml) added the palladium (10 wt. % on activated carbon) (2.13g). This slurry was saturated with H₂ by repeated evacuations and H₂ backfills and then stirred for 16 h under one atmosphere pressure of H₂. Celite (5 g) was added to the reaction, the palladium was filtered off and the filtrate was concentrated under reduced pressure to give 59.45 g (98%) of the saturated ester **2** which was carried on without further purification. To a solution of LiAlH₄ (200.00 ml, 1 M in diethyl ether) at -78°C under argon was added the unsaturated ester **2** in anhydrous THF (400 ml) in a slow stream with vigorous mechanical stirring. Upon warming to 20°C additional THF (600 ml) was added and the reaction was stirred 1 h. The mixture was cooled to 0°C and quenched by the careful, consecutive addition of H₂O (7.60 ml), NaOH (7.60 ml of a 15% aqueous solution), and an additional portion of H₂O (22.80 ml). The solids were filtered off and the filtrate was concentrated under reduced pressure. Subsequent purification by chromatography on SiO₂ with 20-50% EtOAc:hexanes gave 50.93 g (98%) of the alcohol **3** as a pale oil. To a solution of oxalyl chloride (20.65 ml, 41.29 mmol) in anhydrous CH₂Cl₂ (100 ml) at -78°C under argon was added dropwise a solution of DMSO (6.72 g, 86.02 mmol) in CH₂Cl₂ (25 ml).

After mechanical stirring for 15 minutes a solution of the alcohol **3** (6.40 g, 34.41 mmol) in CH₂Cl₂ (80 ml) was added dropwise and the mixture was stirred an additional 15 min at -78°C before adding triethylamine (27.85 g, 275.30 mmol). The reaction was stirred 2 h at 20°C and then quenched with saturated aqueous NaHCO₃. This mixture was extracted CH₂Cl₂ and the combined organic fractions were washed consecutively with H₂O and brine, dried (MgSO₄) and concentrated under reduced pressure. The resulting solids were purified by chromatography on SiO₂ with 20-30% EtOAc:hexanes to give 5.08 g, (79%) of the aldehyde **4** as a white solid. A solution of aldehyde **4** (5.08 g, 27.59 mmol) in EtOH (40 ml) was treated with tosylmethyl isocyanide (TosMIC) (5.15 g, 26.27 mmol) and NaCN (0.13 g, 2.68 mmol) at 20°C for 3 h and then refrigerated. After 2 h refrigeration the solids were filtered off, dissolved in anhydrous MeOH saturated with NH₃ (30 ml) and heated in a sealed tube at 100°C for 3.5 h. The reaction was then concentrated under reduced pressure and the residues were taken up in CHCl₃, washed consecutively with saturated aqueous NaHCO₃ and brine, dried (MgSO₄) and concentrated to a red oil. This residue was further purified by chromatography on SiO₂ with 5-10% MeOH (saturated with NH₃): CH₂Cl₂ to give 1.87 g (31%) of the imidazole 5 as a pink oil. A solution of 5 (0.55 g, 2.48 mmol) in acetone (20 ml) containing HCl (5 N, 0.5 ml) was stirred for 5 h. The reaction was concentrated under reduced pressure, the residues were taken up in H₂O, neutralized to pH 7 with saturated aqueous NaHCO₃ and extracted exhaustively with CHCl₃/isopropyl alcohol (3:1). The combined organic portions were washed consecutively with H₂O and brine, dried (MgSO₄) and concentrated. Chromatography on SiO₂ with 5-10% MeOH (saturated with NH₃): CH₂Cl₂ gave 0.43 g (97%) of the desired ketone **6.** A solution of **6** (0.20 g, 1.11 mmol) in anhydrous DMF (4 ml) under argon was treated with triethylamine (0.14 g, 1.33 mmol) and dimethylsulfamoyl chloride (0.19 g, 1.33 mmol) under argon and stirred 16 h. The solids were filtered off and the filtrate was concentrated at via kugelrohr at 100°C and 0.5 torr. The residues were taken up in CHCl₃ and washed consecutively with H₂O and brine, dried (MgSO₄) and concentrated. Chromatography on SiO₂ with 1-5% MeOH:CH₂Cl₂ gave 0.22 g (69%) of the desired protected imidazole **7** as a mixture of regioisomers which were carried on without separation. A solution of **7** (0.18 g, 0.62 mmol) in anhydrous THF (10 ml) under argon was treated with methylmagnesium chloride (0.32 ml, 3.0 M in THF) and the resulting mixture was stirred 16 h. The reaction was quenched with a small amount of MeOH, concentrated under reduced pressure and the residues were taken up in H₂O. The mixture was acidified by the dropwise addition of 1 N HCl until the solution was homogenious and then the pH was adjusted to **7** with saturated aqueous NaHCO₃. The organic materials were extracted into CHCl₃ and the combined organic portions were washed consecutively with H₂O and brine, dried (MgSO₄) and concentrated. Chromatography on SiO₂ with 5% MeOH:CH₂Cl₂ gave 0.18 g (95%) of the alcohol **8** as a mixture of regioisomers which were carried on without separation. A solution of **8** (0.14 g, 0.46 mmol) in anhydrous benzene (3 ml) at 0°C under argon was treated with (methoxycarbonylsulfamoyl) triethylammonium hydroxide, inner salt (Burgess reagent) (0.12 g, 0.51 mmol) and stirred 1 h at 20°C. The reaction was concentrated under reduced pressure and subsequent purification by chromatography on SiO₂ with 5% MeOH:CH₂Cl₂ gave 0.12 g (92%) of the alkenes **9** and **10** as a mixture of isomers which were carried on without separation. The mixture of isomers **9** and **10** (0.12 g, 0.42 mmol) were refluxed in a solution composed ofMeOH (2 ml) and KOH (2 ml of a 5 N solution) for 30 h. The reaction was concentrated under reduced pressure and the residues were taken up in H₂O and extracted exhaustively with CHCl₃. The combined organic portions were washed consecutively with H₂O and brine, dried (MgSO₄) and concentrated. Chromatography on SiO₂ with 5-10% MeOH (saturated with NH₃): CH₂Cl₂ gave 0.05 g (67%) of alkenes **11** and **12** as a mixture of isomers which were carried on without separation.

The mixture of alkenes **11** and **12** (0.045 g, 0.26 mmol) and p-toluenesulfonic acid hydrate (0.063 g, 0.32 mmol) were heated at reflux in 1,2-dichloroethane (2 ml) under argon for 20 h. The reaction was concentrated under reduced pressure and the residues were purified by chromatography on SiO₂ with 10% MeOH (saturated with NH₃): CH₂Cl₂ to give the free base of imidazole **13** (X-1) as one isomer. The imidazole was recrystallized by stirring in MeOH or THF with an equimolar amount of fumaric acid until all solids had disappeared followed by the addition of a small amount of diethyl ether and cold storage. The title compound **13** (X-1) 0.040 g (54%) was recovered as white crystals.
^{**1**}**H NMR** (300 MHz, DMSO w/ TMS) : δ 7.65 (s, 1 H), 6.78 (s, 1 H), 6.60 (s, 2 H), 5.31 (s, 1 H), 2.44 (d, J = 6.7 Hz, 2 H), 2.02-1.82 (m, 3 H), 1.82-1.60 (m, 3 H), 1.59 (s, 3 H), 1.26-1.11 (m, 1 H)
^{**13**}**C NMR** (75MHz, DMSO-d₆ w/ TMS) : δ 175.0, 165.2, 134.3, 134.1, 133.2, 120.3, 118.3, 33.2, 32.4, 31.2, 29.3, 28.3, 23.4.

### Example X-2

4(5)-(4-Ethyl-cyclohex-3-enylmethyl)-1H-imidazole, but-2-enedioic acid salt is prepared by substituting ethyl magnesium chloride in the method of X-1

### Example X-3

4(S)-(4-Pentyl-cyclohex-3-enylinethyl)-1H-imidazole, but-2-enedioic acid salt is prepared by substituting pentyl magnesium chloride in the method of X-1

### Example Y

A method for measuring α-agonist selectivity comprises the RSAT (Receptor Selection and Amplification Technology) assay as reported in Messier et al. (1995) "High throughput assays of cloned adrenergic, muscarinic, neurokinin and neurotrophin receptors in living mammalian cells", *Pharmacol. Toxicol.* **76**:308-11 and adapted for use with alpha₂ receptors. The assay measures a receptor-mediated loss of contact inhibition that results in selective proliferation of receptor-containing cells in a mixed population of confluent cells. The increase in cell number is assessed with an appropriate transfected marker gene such as b-galactosidase, the activity of which can be easily measured in a 96-well format. Receptors that activate the G protein, G_{q}, elicit this response. Alpha₂ receptors, which normally couple to Gᵢ, activate the RSAT response when coexpressed with a hybrid Gq protein that has a Gᵢ receptor recognition domain, called G_{q/i5}². See Conklin et al. (1993) "Substitution of three amino acids switches receptor specificity of G_{q^{a}} to that of G_{i^{a}}." *Nature* **363**:274-6.

NIH-3T3 cells are plated at a density of 2x10⁶ cells in 15 cm dishes and maintained in Dulbecco's modified Eagle's medium supplemented with 10% calf serum. One day later, cells are cotransfected by calcium phosphate precipitation with mammalian expression plasmids encoding p-SV-b-galactosidase (5-10 mg), receptor (1-2 mg) and G protein (1-2 mg). 40 mg salmon sperm DNA may also be included in the transfection mixture. Fresh media is added on the following day and 1-2 days later, cells are harvested and frozen in 50 assay aliquots. Cells are thawed and 100 ml added to 100 ml aliquots of various concentrations of drugs in triplicate in 96-well dishes. Incubations continue 72-96 hr at 37°. After washing with phosphate-buffered saline, b-galactosidase enzyme activity is determined by adding 200 ml of the chromogenic substrate (consisting of 3.5 mM o-nitrophenyl-b-D-galactopyranoside and 0.5% nonidet P-40 in phosphate buffered saline), incubating overnight at 30° and measuring optical density at 420 nm. The absorbence is a measure of enzyme activity, which depends on cell number and reflects a receptor-mediated cell proliferation. The EC₅₀ and maximal effect of each drug at each alpha₂ receptor is determined. The efficacy or intrinsic activity is calculated as a ratio of the maximal effect of the drug to the maximal effect of a standard full agonist for each receptor subtype. Brimonidine, also called UK14,304-18, is used as the standard agonist for the alpha_{2A} and alpha_{2C} receptors. Oxymetazoline is the standard agonist used for the alpha_{2B} receptor.

Table 1, below, provides the intrinsic activity values at subtypes of the α2-adrenoreceptor as determined in the RSAT assay for the compounds of above Examples S to X-3 and certain adrenergic compounds not having selective agonist activity at the α2B or α2B /α2C subtype(s). At the α2A subtype, the compounds of the Examples are inactive or exhibit low efficacy (≤0.4). They have greater efficacy at the α2B and the α2C- subtypes than the α2A-subtype. Therefore, unlike ophthalmic α2-adrenoreceptor compounds such as clonidine and brimonidine, the compounds of Examples B through X can selectively activate α2-adrenoreceptor subtypes other than the α2A-subtype.

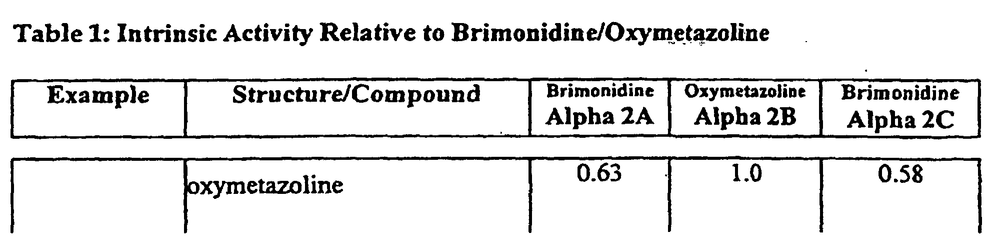

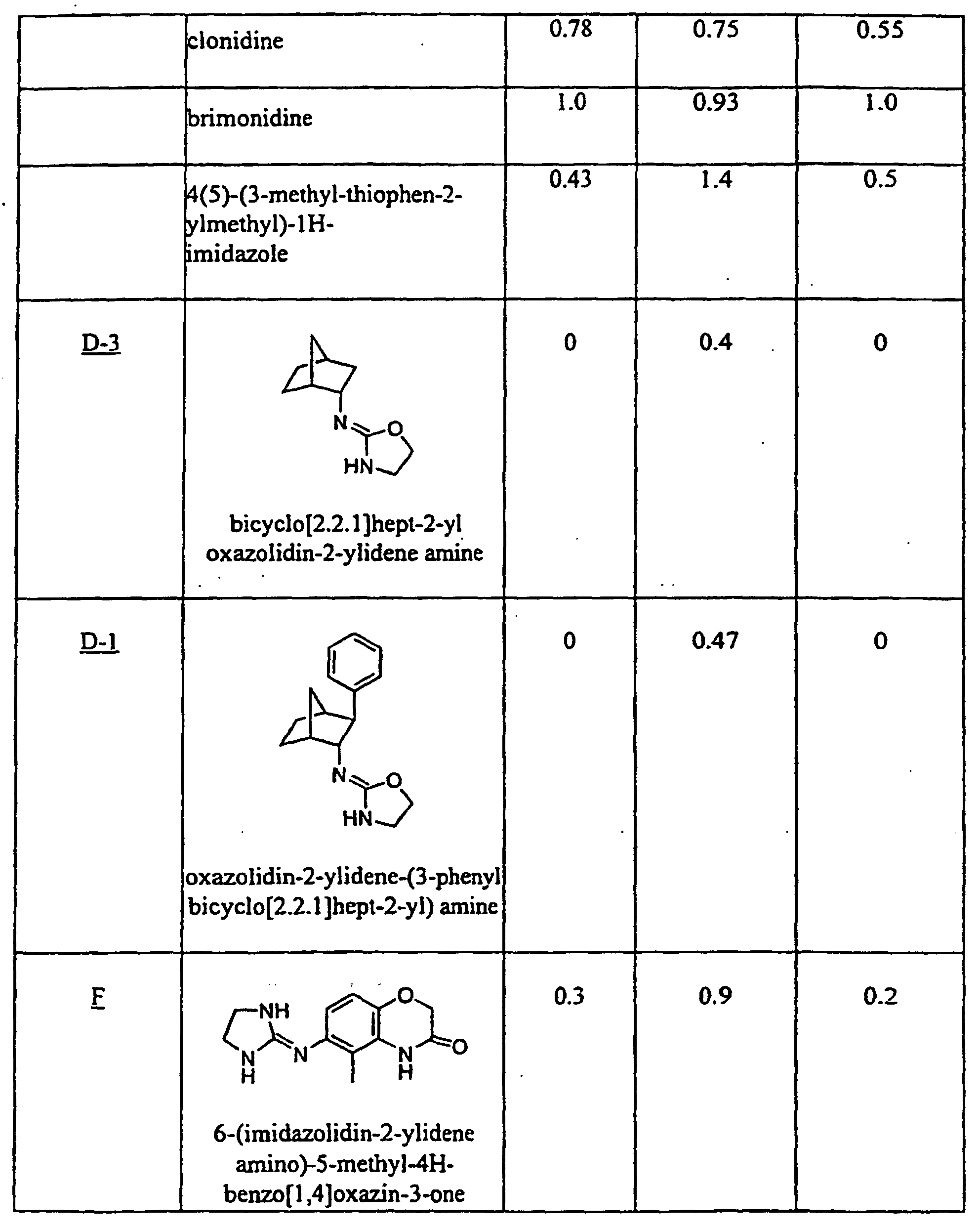

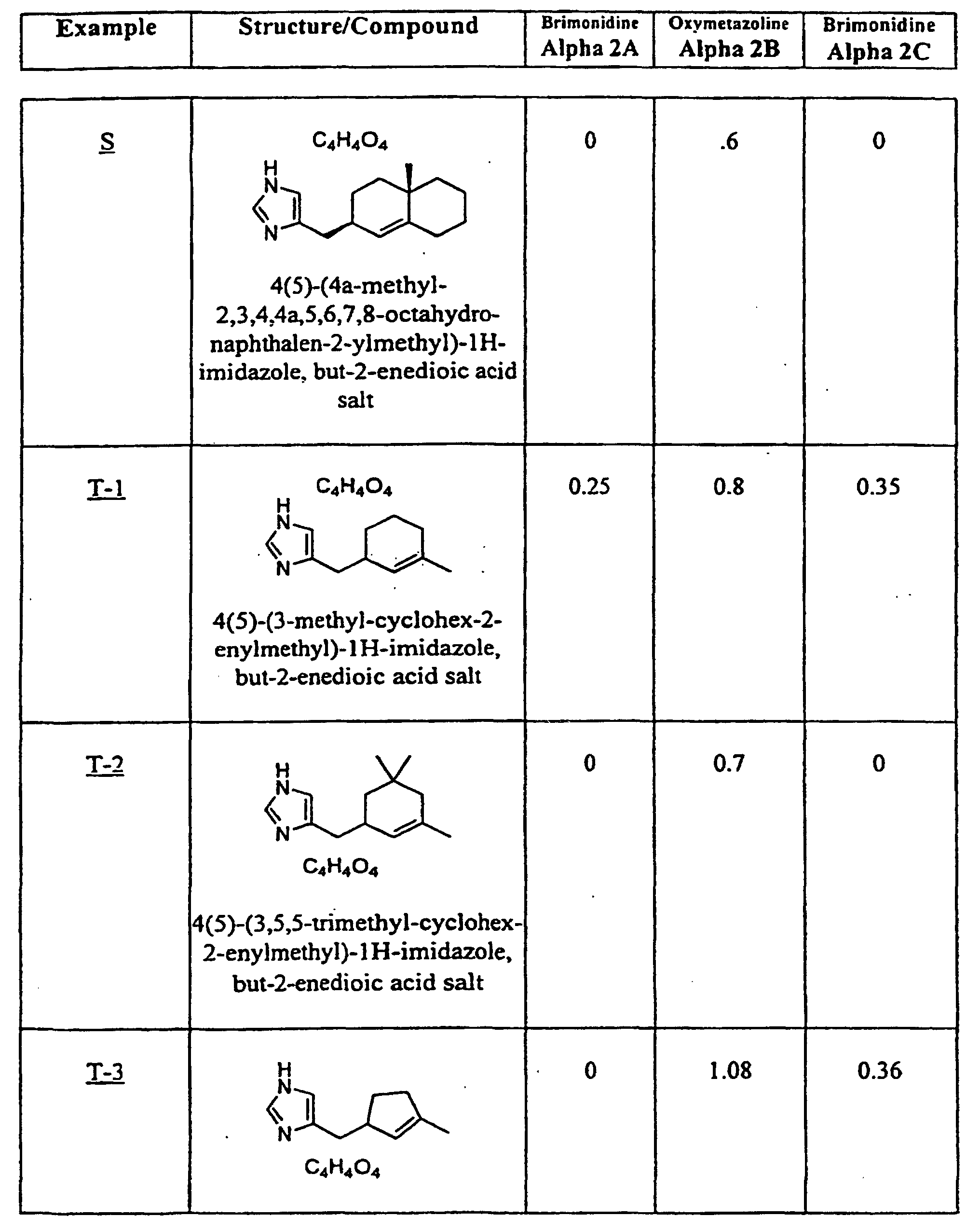

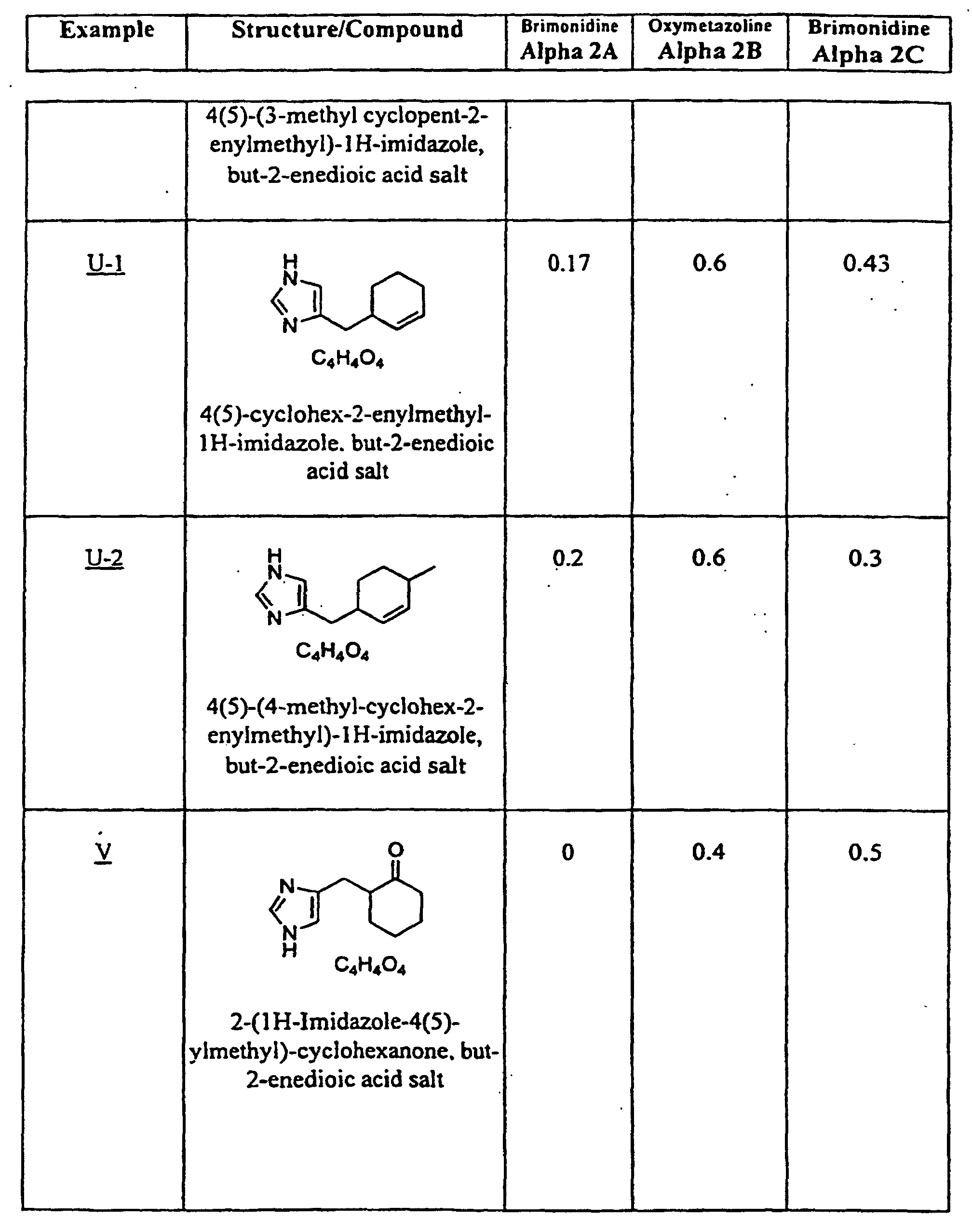

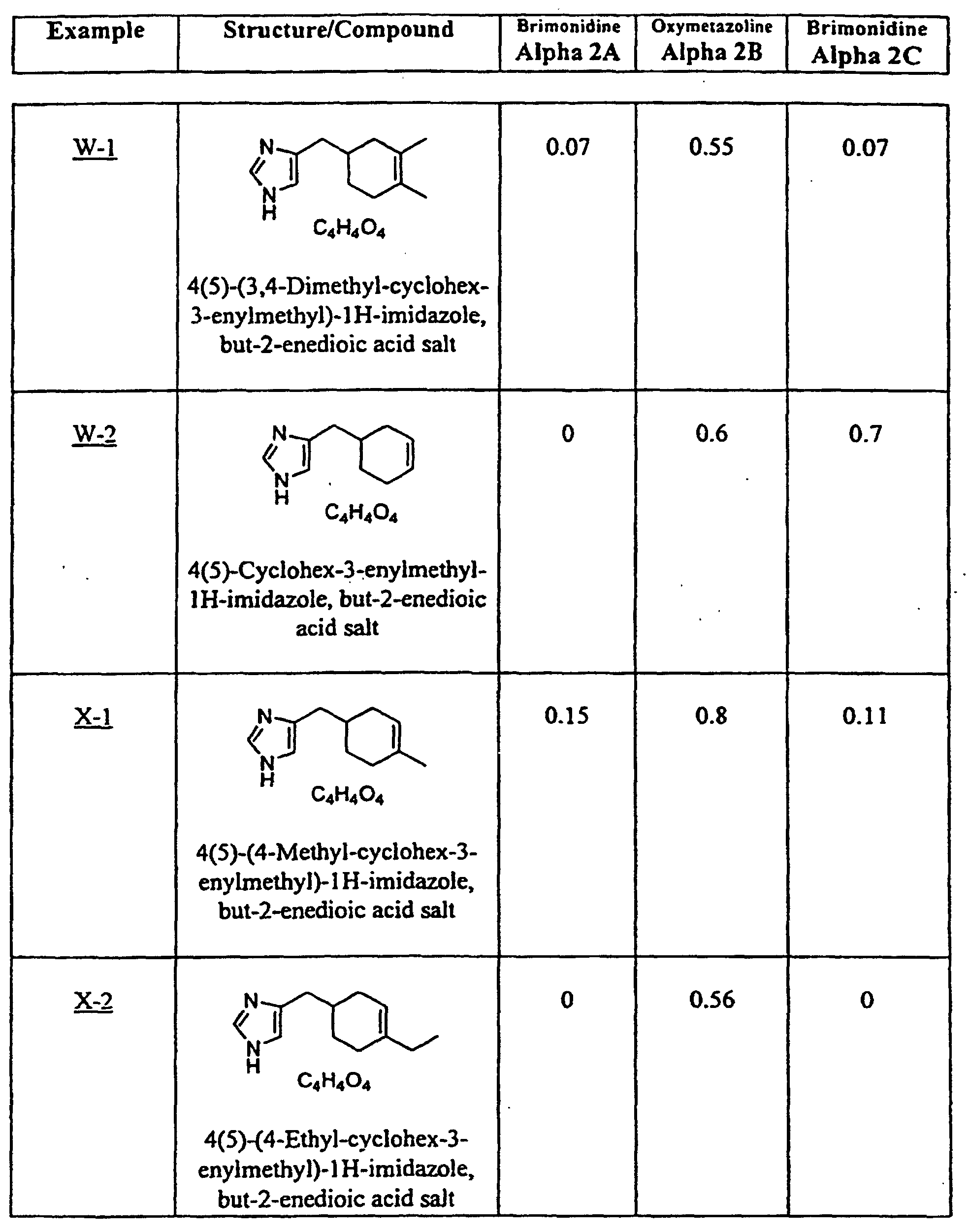

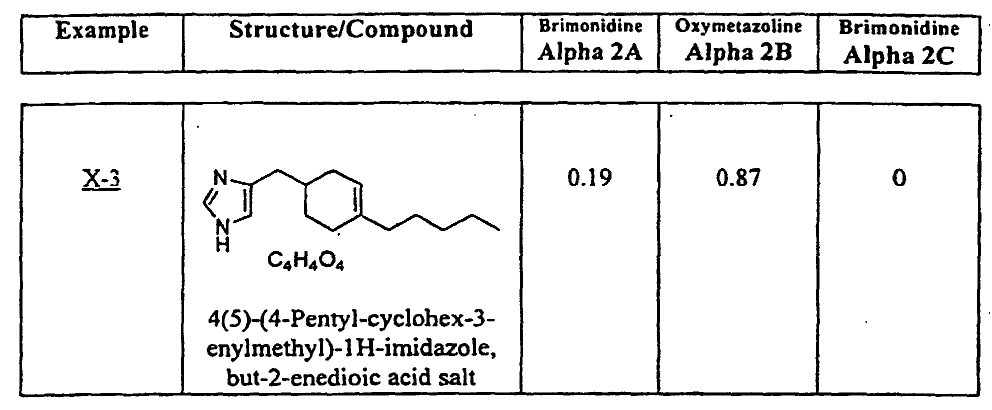

## Claims

1. A compound represented by the formula: 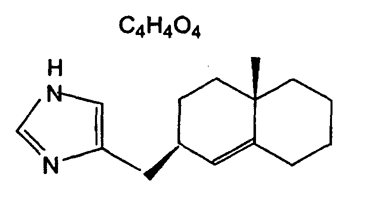 4(5)-(4a-methyl-2,3,4,4a,5,6,7,8-octahydronaphthalen-2-ylmethyl)-1H-imidazole, but -2-enedioic acid salt

2. A compound represented by the formula: 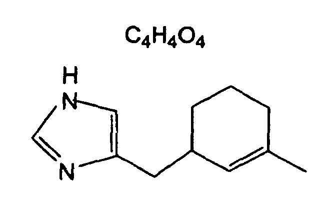 4(5)-(3methyl-cyclohex-2-enylmethyl)-1 H-imidazole, but-2-enedioic acid salt

3. A compound represented by the formula: 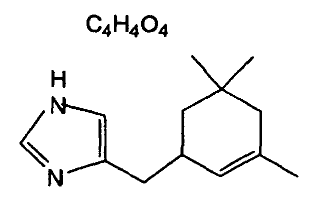 4(5)-(3,5,5-trimethyl-cyclohex-2-enylmethyl)-1H-imidazole, but-2-enedioic acid salt

4. A compound represented by the formula: 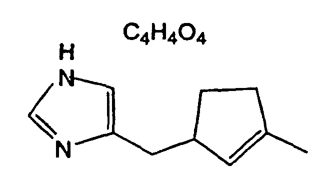 4(5)-(3-methylcyclopent-2-enylmethyl)-1H-imidazole, but-2-enedioic acid salt

5. A compound having selective agonist activity at the α2B or α2B/α2C adrenergic receptor subtype(s) as compared to the adrenergic receptor subtype represented by the formula: 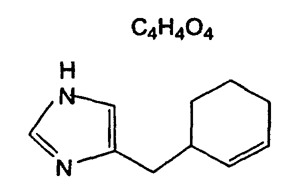 4(5)-cyclohex-2-enylmethyl-1H-imidazole, but-2-enedioic acid salt

6. A compound represented by the formula: 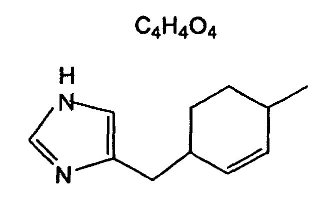 4(5)-(4-methyl-cyclohex-2-enylmethyl-1H-imidazole, but-2-enedioic acid salt

7. A compound represented by the formula: 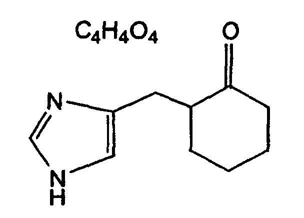 2-(1H-Imidazole-4(5)-ylmethyl)-cyclohexanone, but-2-enedioic acid salt

8. A compound represented by the formula: 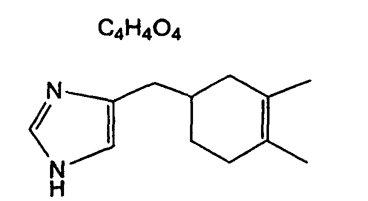 4(5)-(3,4-Dimethyl-cyclohex-3-enylmethyl)-1H-imidazole, but-2-enedioic acid salt

9. A compound represented by the formula: 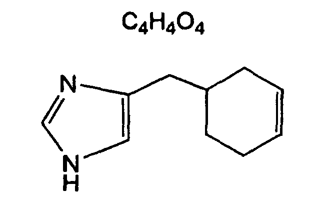 4(5)-Cyclohex-3-enylmethyl-1H-imidazole, but-2-enedioic acid salt

10. A compound represented by the formula: 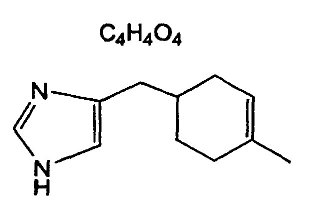 4(5)-(4-Methyl-cyclohex-3-enylmethyl)-1H-imidazole, but-2-enedioic acid salt

11. A compound represented by the formula: 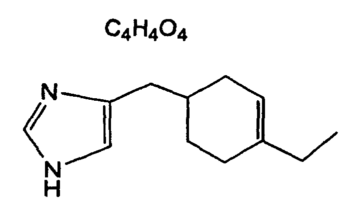 4(5)-(4-Ethyl-cyclohex-3-enylmethyl)-1H-imidazole, but-2-enedioic acid salt

12. A compound represented by the formula: 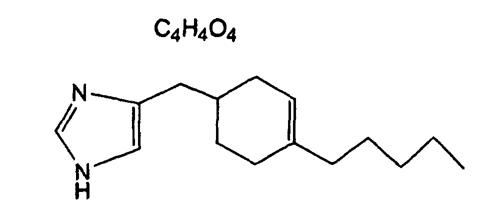 4(5)-(4-Pentyl-cyclohex-3-enylmethyl)-1H-imidazole, but-2-enedioic acid salt

13. Use of a compound according to claims 1 to 12 for the preparation of a medicament
• for the treatment or prevention of glaucoma,
• for the treatment of elevated intraocular pressure without sedating or cardiovascular side effects,
• to lower intraocular pressure without having cardiovascular and sedative side effects.

14. Use of a compound according to claims 1 to 13 for the manufacture of a pharmaceutical composition for the treatment and prevention of
muscle spasticity including hyperactive micturition, diarrhea, diuresis, withdrawal syndromes, pain including neuropathic pain, neurodegenerative diseases including optic neuropathy, spinal ischemia and stroke, memory and cognition deficits, attention deficit disorder, psychoses including manic disorders, anxiety, depression, hypertension, congestive heart failure, cardiac ischemia and nasal congestion without sedating or cardiovascular side effects.

## Patentansprüche

1. Verbindung, dargestellt durch die Formel: 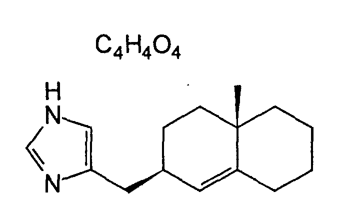 4(5)-(4a-Methyl-2,3,4,4a,5,6,7,8-octahydronaphthalin-2-ylmethyl)-1Himidazol-But-2-endionsäuresalz

2. Verbindung, dargestellt durch die Formel: 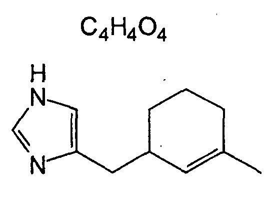 4(5)-(3-Methyl-cyclohex-2-enylmethyl)-1H-imidazol-But-2-endionsäuresalz

3. Verbindung, dargestellt durch die Formel: 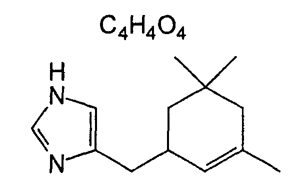 4(5)-(3,5,5-Trimethyl-cyclohex-2-enylmethyl)-1H-imidazol-But-2-endionsäuresalz

4. Verbindung, dargestellt durch die Formel: 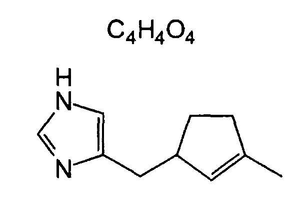 4(5)-(3-Methylcyclopent-2-enylmethyl)-1H-imidazol-But-2-endionsäuresalz

5. Verbindung, die selektive Agonistenaktivität an dem/den α2B- oder α2B/α2C-adrenergischen Rezeptorsubtyp(en) im Vergleich zu dem/den adrenergischen Rezeptorsubtyp(en), dargestellt durch die Formel, aufweist: 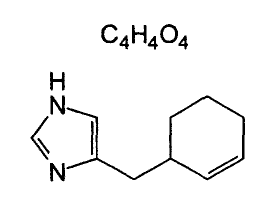 4(5)-Cyclohex-2-enylmethyl-1H-imidazol-But-2-endionsäuresalz

6. Verbindung, dargestellt durch die Formel: 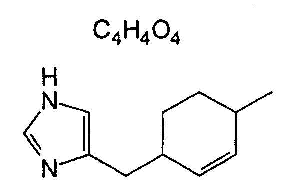 4(5)-(4-Methyl-cyclohex-2-enylmethyl)-1H-imidazol-But-2-endionsäuresalz

7. Verbindung, dargestellt durch die Formel: 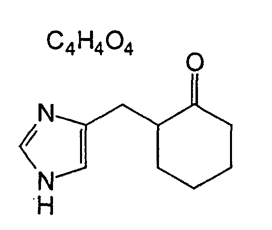 2-(1H-Imidazol-4(5)-ylmethyl)-cyclohexanon-But-2-endionsäuresalz

8. Verbindung, dargestellt durch die Formel: 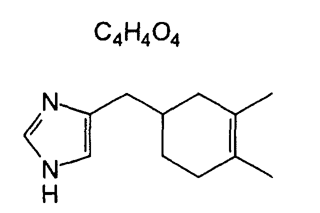 4(5)-(3,4-Dimethyl-cyclohexyl-3-enylmethyl)-1H-imidazol-But-2-endionsäuresalz

9. Verbindung, dargestellt durch die Formel: 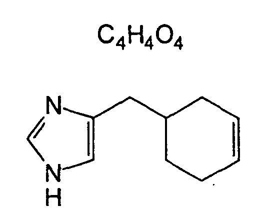 4(5)-Cyclohex-3-enylmethyl-1H-imidazol-But-2-endionsäuresalz

10. Verbindung, dargestellt durch die Formel: 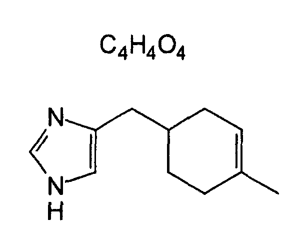 4(5)-(4-Methyl-cyclohex-3-enylmethyl)-1H-imidazol-But-2-endionsäuresalz

11. Verbindung, dargestellt durch die Formel: 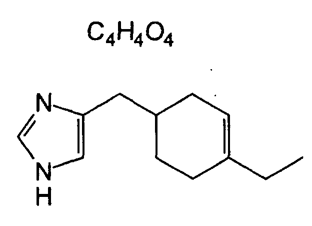 4(5)-(4-Ethyl-cyclohex-3-enylmethyl)-1H-imidazol-But-2-endionsäuresalz

12. Verbindung, dargestellt durch die Formel: 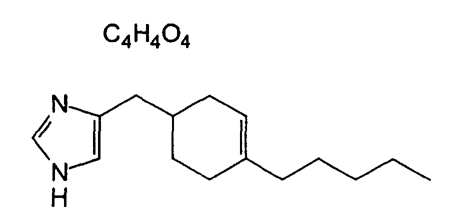 4(5)-(4-Pentyl-cyclohex-3-enylmethyl)-1H-imidazol-But-2-endionsäuresalz

13. Verwendung einer Verbindung nach den Ansprüchen 1 bis 12 zur Herstellung eines Medikaments
• zur Behandlung oder Vorbeugung von Glaukoma,
• zur Behandlung von erhöhtem Intraokulardruck ohne sedative oder kardiovaskuläre Nebenwirkungen,
• um Intraokulardruck ohne kardiovaskuläre und sedative Nebenwirkungen zu verringern.

14. Verwendung einer Verbindung nach den Ansprüchen 1 bis 13 zur Herstellung einer pharmazeutischen Zusammensetzung zur Behandlung und Vorbeugung von Muskelspasmen, einschliesslich hyperaktiver Blasenentleerung, Durchfall, Diurese, Entzugserscheinungen, Schmerzen, einschliesslich neuropathischen Schmerzen, neurodegenerative Schmerzen, einschliesslich optischer Neuropathie, spinaler Ischämie und Schlaganfall, Gedächtnis- und Wahrnehmungsschwäche, Aufmerksamkeitsstörung, Psychosen, einschliesslich manischen Störungen, Angstzustand, Depression, Hypertonie, kongestivem Herzversagen, Herzischämie und nasaler Stauung ohne sedative oder kardiovaskuläre Nebenwirkungen.

## Revendications

1. Composé représenté par la formule : 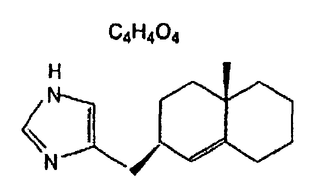 4(S)-(4a-méthyl-2,3,4,4a,5,6,7,8-octahydronaphtalén-2-ylméthyl)-1H-imidazole, sel d'acide but-2-ènedioïque.

2. Composé représenté par la formule : 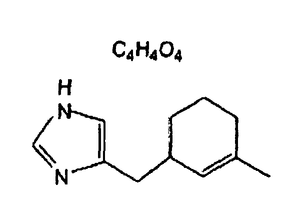 4(5)-(3-méthyl-cyclohex-2-énylméthyl)-1H-imidazole, sel d'acide but-2-ènedioïque.

3. Composé représenté par la formule : 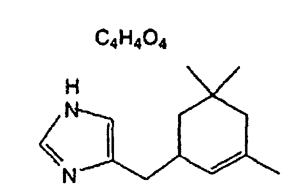 4(5)-(3,5,5-triméthyl-cyclohex-2-énylméthyl)-1H-imidazole, sel d'acide but-2-ènedioïque.

4. Composé représenté par la formule : 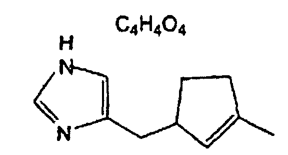 4(5)-(3-méthyl-cyclopent-2-énylméthyl)-1H-imidazole, sel d'acide but-2-ènedioïque.

5. Composé ayant une activité agoniste sélective au(x) sous-type(s) des récepteurs adrénergiques α2B ou α2B/α2C par comparaison au sous-type des récepteurs adrénergiques représenté par la formule : 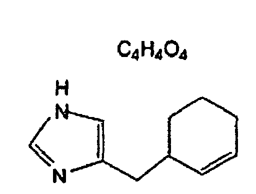 4(5)-cyclohex-2-énylméthyl-1H-imidazole, sel d'acide but-2-ènedioïque.

6. Composé représenté par la formule : 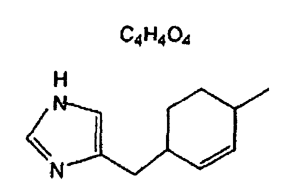 4(5)-(4-méthyl-cyclohex-2-énylméthyl)-1H-imidazole, sel d'acide but-2-ènedioïque.

7. Composé représenté par la formule : 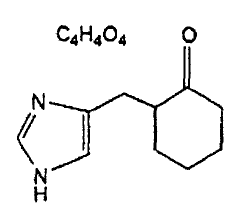 2-(1H-imidazole-4(5)-ylméthyl)cyclohexanone, sel d'acide but-2-ènedioïque.

8. Composé représenté par la formule : 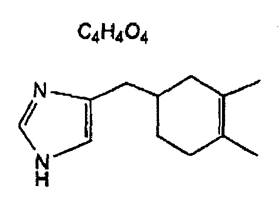 4(5)-(3,4-diméthyl-cyclohex-3-énylméthyl)-1H-imidazole, sel d'acide but-2-ènedioïque.

9. Composé représenté par la formule : 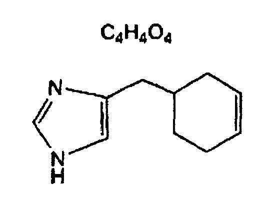 4(5)-cyclohex-3-énylméthyl-1H-imidazole, sel d'acide but-2-ènedioïque.

10. Composé représenté par la formule : 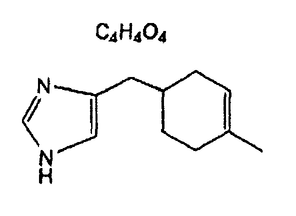 4(5)-(4-méthyl-cyclohex-3-énylméthyl)-1H-imidazole, sel d'acide but-2-ènedioïque.

11. Composé représenté par la formule : 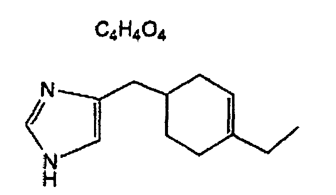 4(5)-(4-éthyl-cyclohex-3-énylméthyl)-1H-imidazole, sel d'acide but-2-ènedioïque.

12. Composé représenté par la formule : 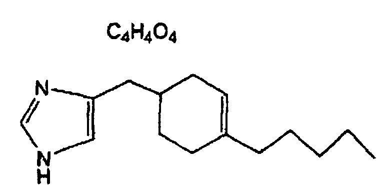 4(5)-(4-pentyl-cyclohex-3-énylméthyl)-1H-imidazole, sel d'acide but-2-ènedioïque.

13. Utilisation d'un composé selon les revendications 1 à 12 pour la préparation d'un médicament destiné
au traitement ou à la prévention du glaucome,
au traitement d'une pression intra-oculaire élevée sans effets secondaires sédatifs ou cardiovasculaires,
à abaisser la pression intra-oculaire sans avoir d'effets secondaires sédatifs ou cardiovasculaires.

14. Utilisation d'un composé selon les revendications 1 à 13 pour la fabrication d'une composition pharmaceutique destinée au traitement et à la prévention de la spasticité musculaire y compris la miction hyperactive, les diarrhées, la diurèse, les syndromes de manque, les douleurs y compris les douleurs névropathiques, les maladies neurodégénératives y compris la neuropathie optique, l'ischémie spinale et les attaques, les déficiences de la mémoire et cognitives, le trouble de l'inattention, les psychoses y compris les troubles maniaques, l'anxiété, la dépression, l'hypertension, l'insuffisance cardiaque congestive, l'ischémie cardiaque et la congestion nasale sans effets secondaires sédatifs ou cardiovasculaires.
